# EUROPEAN PATENT APPLICATION

(11) **EP 4 563 092 A1**
(43) Date of publication of application: **04.06.2025**
(21) Application number: 22953151.2
(22) Date of filing: 28.07.2022
(51) Int. Cl.: A61B 10/00

(54) **SYMPTOM DETECTION PROGRAM, SYMPTOM DETECTION METHOD, AND SYMPTOM DETECTION DEVICE**

(71) Applicant: FUJITSU LIMITED, Kawasaki-shi, Kanagawa 211-8588 (JP)
(72) Inventor: YOUOKU, Sachihiro, Kawasaki-shi, Kanagawa 211-8588 (JP); MI, Xiaoyu, Kawasaki-shi, Kanagawa 211-8588 (JP)
(74) Representative: Hoffmann Eitle
(86) International application number: PCT/JP2022/029199
(87) International publication number: WO 2024/024062

(57) **Abstract**

A symptom detection device acquires video data including a face of a patient who is executing a specific task. The symptom detection device detects each occurrence intensity of each action unit included in the face of the patient, by analyzing the acquired video data. The symptom detection device detects a symptom related to a major neurocognitive disorder of the patient, based on a temporal change in the detected occurrence intensity of each of the plurality of action units.

## Description

### TECHNICAL FIELD

The present invention relates to a symptom detection program, a symptom detection method, and a symptom detection device.

### BACKGROUND ART

Typically, it has been known that a specialized doctor diagnoses a major neurocognitive disorder that makes a person be unable to perform basic actions such as eating meals or bathing or a mild cognitive impairment that makes a person be unable to perform complicated actions such as shopping or household matters although the basic operations can be performed, from computed tomography (CT), blood tests, or the like.

### CITATION LIST

### PATENT DOCUMENT

Patent Document 1: Japanese Laid-open Patent Publication No. 2022-61587

### SUMMARY OF INVENTION

### TECHNICAL PROBLEM

However, diagnosis of an initial major neurocognitive disorder or a mild cognitive impairment is difficult, because symptoms are less likely to appear from typical CT, blood tests, or the like. For example, at the time of emergency diagnosis such as emergency transport or a nighttime outpatient, a doctor other than a specialist may make a diagnosis, and there is an increasing possibility that erroneous diagnosis or the like occurs.

In one aspect, an object is to provide a symptom detection program, a symptom detection method, and a symptom detection device that can detect a symptom related to a major neurocognitive disorder early.

### SOLUTION TO PROBLEM

In a first idea, a symptom detection program causes a computer to execute processing including acquiring video data that includes a face of a patient who is executing a specific task, detecting each occurrence intensity of each action unit included in the face of the patient, by analyzing the acquired video data, and detecting a symptom related to a major neurocognitive disorder of the patient, based on a temporal change in the detected occurrence intensity of each of the plurality of action units.

### ADVANTAGEOUS EFFECTS OF INVENTION

According to one embodiment, it is possible to detect a symptom related to a major neurocognitive disorder early.

### BRIEF DESCRIPTION OF DRAWINGS

FIG. 1 is a diagram for explaining a symptom detection device according to a first embodiment.
FIG. 2 is a diagram for explaining a reference technique.
FIG. 3 is a functional block diagram illustrating a functional configuration of the symptom detection device according to the first embodiment.
FIG. 4 is a diagram for explaining a generation example of a first machine learning model.
FIG. 5 is a diagram illustrating a camera arrangement example.
FIGs. 6A-6C are diagrams for explaining a movement of a marker.
FIG. 7 is a diagram for explaining training of a second machine learning model.
FIGs. 8A-8C are diagrams illustrating an example of a specific task.
FIG. 9 is a diagram for explaining generation of training data of the second machine learning model.
FIG. 10 is a diagram for explaining detection of a mild cognitive impairment.
FIG. 11 is a diagram for explaining details of the detection of the mild cognitive impairment.
FIG. 12 is a flowchart illustrating a flow of preprocessing.
FIG. 13 is a flowchart illustrating a flow of detection processing.
FIG. 14 is a diagram for explaining another example of the training data of the second machine learning model.
FIG. 15 is a diagram for explaining an example of a use form of a symptom detection application.
FIG. 16 is a diagram for explaining a hardware configuration example.

### DESCRIPTION OF EMBODIMENTS

Hereinafter, embodiments of a symptom detection program, a symptom detection method, and a symptom detection device according to the present invention will be described in detail with reference to the drawings. Note that the present invention is not limited by these embodiments. In addition, the embodiments may be appropriately combined with each other in a range without inconsistency.

### [First Embodiment]

### <Overall Configuration>

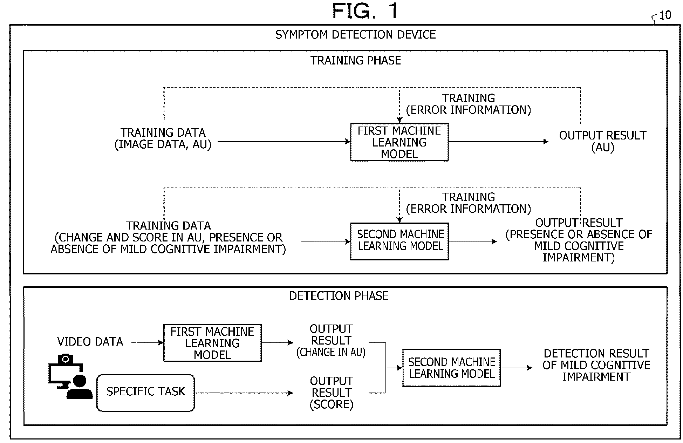

FIG. 1 is a diagram for explaining a symptom detection device 10 according to a first embodiment. The symptom detection device 10 illustrated in FIG. 1 is an example of a computer that finds a major neurocognitive disorder or a mild cognitive impairment early, using a facial expression recognition technology.

In medical fields, a specialized doctor diagnoses a major neurocognitive disorder that makes a person be unable to perform basic actions such as eating meals or bathing or a mild cognitive impairment that makes a person be unable to perform complicated actions such as shopping or household matters although the basic operations can be performed, from CT, blood tests, or the like.

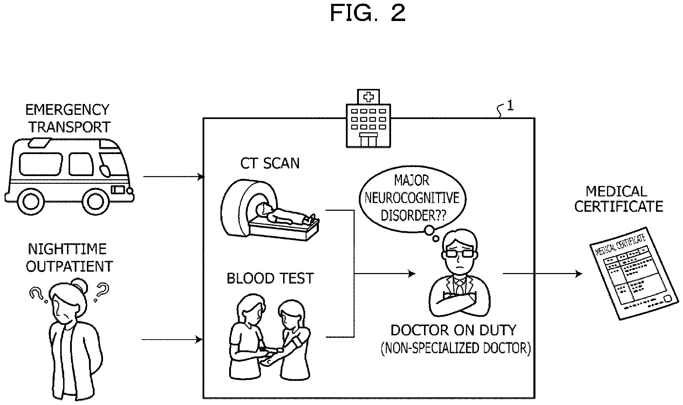

FIG. 2 is a diagram for explaining a reference technique. As illustrated in FIG. 2, in a case where a patient comes to a hospital 1 for an examination by emergency transport or as a nighttime outpatient, a doctor on duty in the hospital 1 makes a diagnosis using the CT or the blood test. However, since symptoms of an initial major neurocognitive disorder or a mild cognitive impairment hardly appear from the examination or the like, it is difficult for a doctor other than the specialist to diagnose. Therefore, in a case where the doctor on duty is not the specialist, there is an increasing possibility that erroneous diagnosis or the like occurs and this adversely affects on a subsequent treatment.

Therefore, the symptom detection device 10 according to the first embodiment realizes early detection of the major neurocognitive disorder or the mild cognitive impairment, using video data at the time when the patient executes a specific task (application) to apply a load to a cognitive function so as to examine the cognitive function. Note that, in the present embodiment, an example will be described in which the symptom detection device 10 executes both of the specific task and the symptom detection. However, the specific task and the symptom detection can be executed by separate devices.

Specifically, the symptom detection device 10 generates each machine learning model used to detect the symptoms of the major neurocognitive disorder or the mild cognitive impairment, in a training phase. For example, as illustrated in FIG. 1, the symptom detection device 10 generates a first machine learning model that outputs an intensity of each action unit (AU) from image data in the training phase and a second machine learning model that outputs a result of detecting whether or not the mild cognitive impairment occurs from a temporal change in the AU and a score of the specific task.

More specifically, the symptom detection device 10 generates the first machine learning model by inputting training data using image data in which a face of the patient is imaged as an explanatory variable and an occurrence intensity (value) of each AU as an objective variable into the first machine learning model and training a parameter of the first machine learning model so as to minimize error information between an output result of the first machine learning model and the objective variable.

Furthermore, the symptom detection device 10 generates the second machine learning model by inputting training data using the explanatory variable including the temporal change in the occurrence intensity of each AU when the patient is executing the specific task and the score that is an execution result of the specific task and whether or not the mild cognitive impairment occurs as the objective variable into the second machine learning model and training a parameter of the second machine learning model, so as to minimize error information between an output result of the second machine learning model and the objective variable.

Thereafter, the symptom detection device 10 detects whether or not a symptom related to the major neurocognitive disorder occurs, using the video data at the time when the patient executes the specific task and each trained machine learning model, in a detection phase.

For example, as illustrated in FIG. 1, the symptom detection device 10 acquires the video data of the patient who executes the specific task, inputs each frame (image data) in the video data into the first machine learning model as a feature amount, and acquires the occurrence intensity of each AU for each frame. In this way, the symptom detection device 10 acquires a change (change pattern) in the occurrence intensity of each AU of the patient who executes the specific task. Furthermore, the symptom detection device 10 acquires the score of the specific task, after the specific task has been completed. Thereafter, the symptom detection device 10 inputs the temporal change in the occurrence intensity of each AU of the patient and the score into the second machine learning model as feature amounts and acquires a result of detecting whether or not the mild cognitive impairment occurs.

In this way, the symptom detection device 10 can capture a fine change in a facial expression with a less individual difference, by using the AU and can detect the mild cognitive impairment early. Note that, here, as an example of the symptom related to the major neurocognitive disorder of the patient, the mild cognitive impairment has been described. However, the present embodiment is not limited to this and can be similarly applied to other symptoms of the major neurocognitive disorder or the like by setting the objective variable.

### <Functional Configuration>

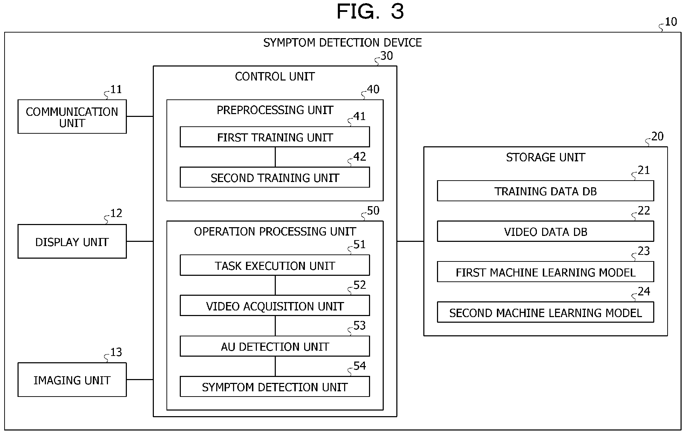

FIG. 3 is a functional block diagram illustrating a functional configuration of the symptom detection device 10 according to the first embodiment. As illustrated in FIG. 3, the symptom detection device 10 includes a communication unit 11, a display unit 12, an imaging unit 13, a storage unit 20, and a control unit 30.

The communication unit 11 is a processing unit that controls communication with another device and, for example, is implemented by a communication interface or the like. For example, the communication unit 11 receives the video data or the score of the specific task to be described later and transmits a processing result to a destination specified in advance, by the control unit 30 to be described later.

The display unit 12 is a processing unit that displays and outputs various types of information, and is implemented by, for example, a display, a touch panel, or the like. For example, the display unit 12 outputs the specific task and receives an answer to the specific task.

The imaging unit 13 is a processing unit that captures a video and acquires video data and is implemented by, for example, a camera or the like. For example, the imaging unit 13 captures a video including the face of the patient while the patient is executing the specific task and stores the video in the storage unit 20 as the video data.

The storage unit 20 is a processing unit that stores various types of data, programs executed by the control unit 30, and the like and, for example, is implemented by a memory, a hard disk, or the like. The storage unit 20 stores a training data DB 21, a video data DB 22, a first machine learning model 23, and a second machine learning model 24.

The training data DB 21 is a database that stores various types of training data used to generate the first machine learning model 23 and the second machine learning model 24. The training data stored here can include supervised training data to which correct answer information is added and unsupervised training data to which the correct answer information is not added.

The video data DB 22 is a database that stores the video data captured by the imaging unit 13. For example, the video data DB 22 stores the video data including the face of the patient while the patient is executing the specific task, for each patient. Note that the video data includes a plurality of time-series frames. A frame number is given to each frame in a time-series ascending order. One frame is image data of a still image captured by the imaging unit 13 at a certain timing.

The first machine learning model 23 is a machine learning model that outputs the occurrence intensity of each AU, according to an input of each frame (image data) included in the video data. Specifically, the first machine learning model 23 estimates an AU that is a method for decomposing and quantifying a facial expression based on a portion of a face and facial expression muscles. This first machine learning model 23 outputs a facial expression recognition result such as "AU 1 : 2, AU 2 : 5, AU 3 : 1,..." that expresses an occurrence intensity (for example, five-step evaluation) of each AU from an AU 1 to an AU 28 set to specify the facial expression, in response to an input of the image data. For example, for the first machine learning model 23, various algorithms such as a neural network or a random forest can be adopted.

The second machine learning model 24 is a machine learning model that outputs whether or not the mild cognitive impairment occurs, according to an input of the feature amount. For example, the second machine learning model 24 outputs a detection result including whether or not the mild cognitive impairment occurs, according to the input of the feature amount including the temporal change (change pattern) in the occurrence intensity of each AU and the score of the specific task. For example, for the second machine learning model 24, various algorithms such as a neural network or a random forest can be adopted.

The control unit 30 is a processing unit that takes overall control of the symptom detection device 10 and is implemented by, for example, a processor or the like. This control unit 30 includes a preprocessing unit 40 and an operation processing unit 50. Note that the preprocessing unit 40 and the operation processing unit 50 are implemented by an electronic circuit included in a processor, a process executed by a processor, or the like.

### (Preprocessing Unit 40)

The preprocessing unit 40 is a processing unit that generates each model, using the training data stored in the storage unit 20, prior to an operation for detecting the symptom related to the major neurocognitive disorder. The preprocessing unit 40 includes a first training unit 41 and a second training unit 42.

The first training unit 41 is a processing unit that generates the first machine learning model 23, by performing training using the training data. Specifically, the first training unit 41 generates the first machine learning model 23, through supervised training using the training data with the correct answer information (label).

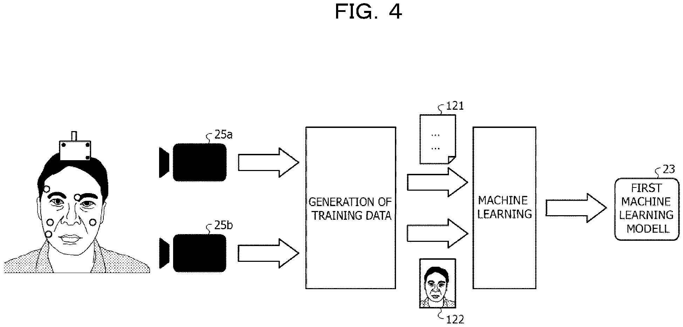

Here, the generation of the first machine learning model 23 will be described with reference to FIGs. 4 to 6A-6C. FIG. 4 is a diagram for explaining a generation example of the first machine learning model 23. As illustrated in FIG. 4, the first training unit 41 generates training data and performs machine learning on image data captured by each of a red-green-blue (RGB) camera 25a and an infrared rays (IR) camera 25b.

As illustrated in FIG. 4, first, the RGB camera 25a and the IR camera 25b are directed to a face of a person to which a marker is attached. For example, the RGB camera 25a is a general digital camera and receives visible light to generate an image. In addition, for example, the IR camera 25b senses infrared rays. Furthermore, the marker is, for example, an IR reflection (retroreflection) marker. The IR camera 25b is capable of performing motion capture by using the IR reflection by the markers. In addition, in the following description, a person to be imaged will be referred to as a subject.

In training data generation processing, the first training unit 41 acquires the image data captured by the RGB camera 25a and a result of the motion capture by the IR camera 25b. Then, the first training unit 41 generates an AU occurrence intensity 121 and image data 122 obtained by deleting a marker from the captured image data through image processing. For example, the occurrence intensity 121 may be data in which the occurrence intensity of each AU is expressed with the five-step evaluation from A to E and annotation is performed as "AU 1 : 2, AU 2 : 5, AU 3 : 1,...".

In machine learning processing, the first training unit 41 performs machine learning, using the image data 122 and the AU occurrence intensity 121 output from the training data generation processing, and generates the first machine learning model 23 used to estimate the AU occurrence intensity from the image data. The first training unit 41 can use the AU occurrence intensity as a label.

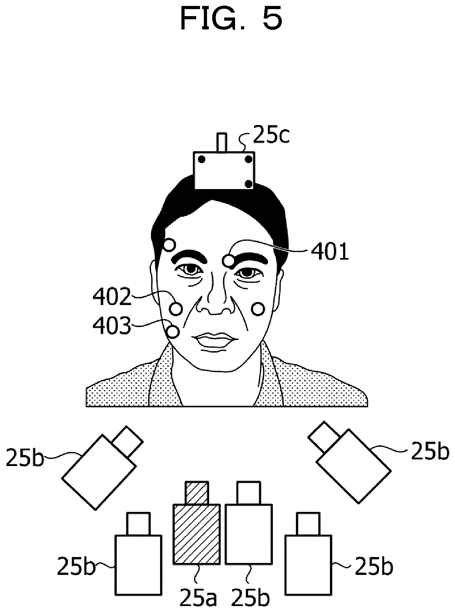

Here, camera arrangement will be described with reference to FIG. 5. FIG. 5 is a diagram illustrating a camera arrangement example. As illustrated in FIG. 5, the plurality of IR cameras 25b may form a marker tracking system. In that case, the marker tracking system may detect a position of the IR reflection marker by stereo imaging. In addition, it is assumed that a relative positional relationship between each of the plurality of IR cameras 25b be corrected in advance through camera calibration.

Furthermore, the plurality of markers is attached to the face of the subject to be imaged so as to cover the AU 1 to the AU 28. Positions of the markers change according to a change in a facial expression of the subject. For example, a marker 401 is arranged near the root of the eyebrow. In addition, a marker 402 and a marker 403 are arranged near the nasolabial line. The markers may be arranged on the skin corresponding to movements of one or more AUs and facial expression muscles. Furthermore, the markers may be arranged to exclude a position on the skin where a texture change is larger due to wrinkles or the like.

Moreover, the subject wears an instrument 25c to which a reference point marker is attached outside a contour of the face. It is assumed that a position of the reference point marker attached to the instrument 25c do not change even when the facial expression of the subject changes. Accordingly, the first training unit 41 is enabled to detect a positional change of the markers attached to the face, based on a change in a relative position from the reference point marker. Furthermore, by setting the number of the reference point markers to three or more, the first training unit 41 can specify a position of the marker in a three-dimensional space.

The instrument 25c is, for example, a headband. In addition, the instrument 25c may be a virtual reality (VR) headset, a mask made of a hard material, or the like. In that case, the first training unit 41 can use a rigid surface of the instrument 25c as the reference point marker.

Note that, when the IR camera 25b and the RGB camera 25a perform imaging, the subject changes his or her facial expression. This enables to acquire, as an image, how the facial expression changes as time passes. In addition, the RGB camera 25a may capture a moving image. The moving image may be regarded as a plurality of still images arranged in time series. Furthermore, the subject may change the facial expression freely, or may change the facial expression according to a predefined scenario.

Note that the AU occurrence intensity can be determined according to a marker movement amount. Specifically, the first training unit 41 can determine an occurrence intensity, based on the marker movement amount calculated based on a distance between a position preset as a determination criterion and the position of the marker.

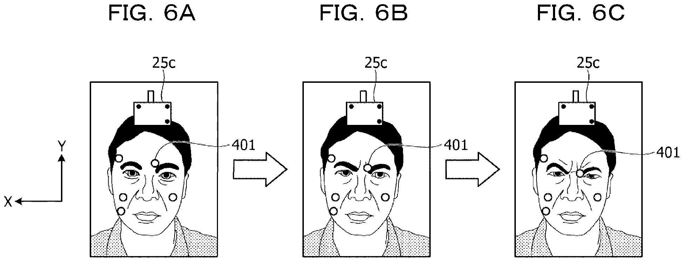

Here, a movement of the marker will be described with reference to FIGs. 6A-6C. FIGs. 6A-6C are diagrams for explaining the movement of the marker. FIGs. 6A to 6C are images captured by the RGB camera 25a. In addition, it is assumed that the images be captured in order of FIG. 6A, FIG. 6B, and FIG. 6C. For example, FIG. 6A is an image when the subject is expressionless. The first training unit 41 may regard positions of the markers in the image FIG. 6A as reference positions where the movement amount is zero. As illustrated in FIGs. 6A-6C, the subject has a facial expression of drawing his/her eyebrows. At this time, the position of the marker 401 moves downward as the facial expression changes. At that time, the distance between the position of the marker 401 and the reference marker attached to the instrument 25c increases.

In this way, the first training unit 41 specifies image data in which a certain facial expression of the subject is imaged and an intensity of each marker at the time of that facial expression and generates training data having an explanatory variable "image data" and an objective variable "an intensity of each marker". Then, the first training unit 41 generates the first machine learning model 23 through supervised training using the generated training data. For example, the first machine learning model 23 is a neural network. The first training unit 41 changes a parameter of the neural network by performing machine learning of the first machine learning model 23. The first training unit 41 inputs the explanatory variable into the neural network. Then, the first training unit 41 generates a machine learning model of which the parameter of the neural network has been changed so as to reduce an error between an output result output from the neural network and correct answer data that is an objective variable.

Note that the generation of the first machine learning model 23 is merely an example, and another method can be used. Furthermore, as the first machine learning model 23, a model disclosed in Japanese Laid-open Patent Publication No. 2021-111114 may be used. Furthermore, a direction of the face can be trained by a similar method.

The second training unit 42 is a processing unit that generates the second machine learning model 24, by performing training using the training data. Specifically, the second training unit 42 generates the second machine learning model 24, through supervised training using training data to which the correct answer information (label) is added.

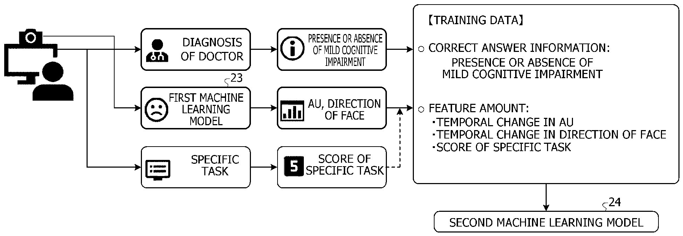

FIG. 7 is a diagram for explaining training of the second machine learning model 24. As illustrated in FIG. 7, the second training unit 42 can train the second machine learning model 24 using the training data prepared in advance or the training data generated using the video data when the patient is executing the specific task and the trained first machine learning model 23.

For example, the second training unit 42 acquires "presence or absence of mild cognitive impairment" as a diagnosis result of the doctor for the patient. Furthermore, the second training unit 42 acquires the occurrence intensity of each AU and the direction of the face obtained by inputting the score that is the result of executing the specific task by the patient and the video data including the face of the patient imaged while the patient is executing the specific task, into the first machine learning model 23.

Then, the second training unit 42 generates training data including "presence or absence of mild cognitive impairment" as "correct answer information" and "temporal change in occurrence intensity of each AU, temporal change in direction of face, and score of specific task" as "feature amounts". Then, the second training unit 42 inputs the feature amount of the training data into the second machine learning model 24 and updates the parameter of the second machine learning model 24, so as to reduce an error between an output result of the second machine learning model 24 and the correct answer information.

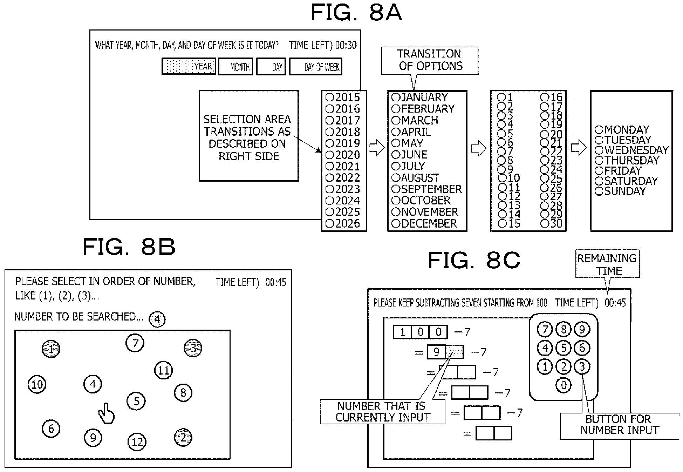

Here, the specific task will be described. FIGs. 8A-8C is a diagram illustrating an example of the specific task. The specific task illustrated in FIGs. 8A-8C is an example of an application or an interactive application that applies a load to a cognitive function and examines the cognitive function.

For example, the specific task illustrated in FIG. 8A is a task for causing the patient to select a date of today. The selection is made by using radio buttons, and a year, a month, a day, a day of week are selected in order from the year. When the answer ends or when it runs over a limit time, the task ends. An answer end time and an answer are registered as scores. Note that, in a case where the time has expired, an answer at the middle and the limit time are used as the scores.

The specific task illustrated in FIG. 8B is a task in which numbers are randomly arranged and displayed and the numbers are selected in order from "1". Clicking one enables to click two, and clicking two enables to click three. The selected number is displayed in a different color, and a number that is currently searched and a remaining time are displayed outside a frame of the task. When the displayed number is XX, XX numbers have been completed. However, when it runs over a limit time YY seconds, the task ends. An end time and the number of achievements (the number of correct answers) are registered as scores.

The specific task illustrated in FIG. 8C is a task in which 100 is displayed and seven is subtracted in order. An item currently input is displayed in a different color, and when the maximum number (XX) of calculations have been completed, the task ends. When XX times of calculations have been completed or when it runs over the limit time YY seconds, the task ends. An end time and an answer are registered as scores. Note that, in a case where the time has expired, an answer at the middle and the limit time are used as the scores.

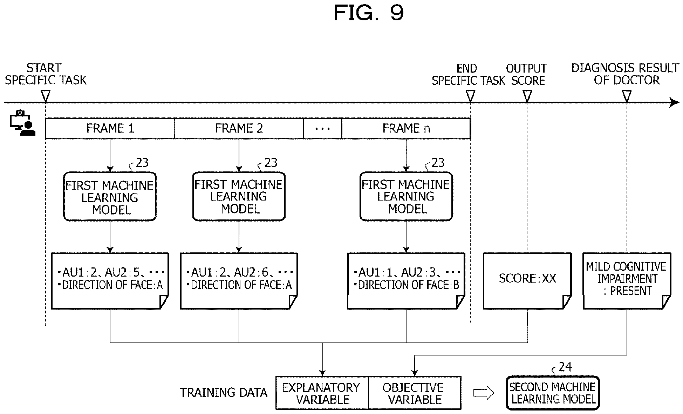

Next, the generation of the training data will be described in detail. FIG. 9 is a diagram for explaining the generation of the training data of the second machine learning model 24. As illustrated in FIG. 9, the second training unit 42 acquires video data imaged from start of the specific task to end of the specific task from a camera or the like and acquires "occurrence intensity of each AU" and "direction of face" from each frame of the video data.

For example, the second training unit 42 inputs image data in a frame 1 into the trained first machine learning model 23 and acquires "AU1 : 2, AU 2 : 5..." and "direction of face: A". Similarly, the second training unit 42 inputs image data in a frame 2 into the trained first machine learning model 23 and acquires "AU1 : 2, AU 2 : 6..." and "direction of face: A". In this way, the second training unit 42 specifies a temporal change in each AU of the patient and a temporal change in the direction of the face of the patient, from the video data.

Furthermore, the second training unit 42 acquires a score "XX" output after the specific task ends. Furthermore, the second training unit 42 acquires a diagnosis result of the doctor for the patient who has executed the specific task "mild cognitive impairment: present", from the doctor, an electronic chart, or the like.

Then, the second training unit 42 generates training data using "occurrence intensity of each AU", "direction of face", and "score (XX)" acquired using each frame as the explanatory variables and "mild cognitive impairment: present" as the objective variable and generates the second machine learning model 24. That is, the second machine learning model 24 trains a relationship between "change pattern of temporal change in occurrence intensity of each AU, change pattern of temporal change in direction of face, and score" and "whether or not mild cognitive impairment occurs".

### (Operation Processing Unit 50)

Returning to FIG. 3, the operation processing unit 50 is a processing unit that includes a task execution unit 51, a video acquisition unit 52, an AU detection unit 53, and a symptom detection unit 54 and detects whether or not mild cognitive impairment of a person (patient) imaged in the video data occurs, using each model prepared by the preprocessing unit 40 in advance.

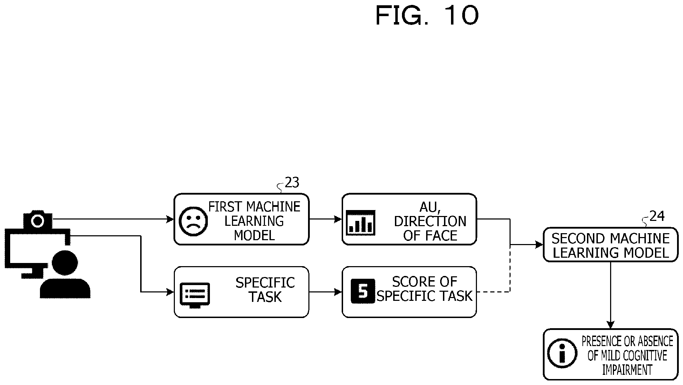

Here, symptom detection will be described with reference to FIG. 10. FIG. 10 is a diagram for explaining detection of the mild cognitive impairment. As illustrated in FIG. 10, the operation processing unit 50 inputs the video data including the face of the patient who is executing the specific task into the trained first machine learning model 23 and specifies the temporal change in each AU of the patient and the temporal change in the direction of the face of the patient. Furthermore, the operation processing unit 50 acquires the score of the specific task. Then, the operation processing unit 50 inputs the temporal change in the AU, the temporal change in the direction of the face, and the score into the second machine learning model 24 and detects whether or not the mild cognitive impairment occurs.

The task execution unit 51 is a processing unit that executes the specific task on the patient and acquires the score. For example, the task execution unit 51 displays any one of the tasks illustrated in FIGs. 8A-8C on the display unit 12 and receives an answer (input) from the patient so as to execute the specific task. Thereafter, the task execution unit 51 acquires the score when the specific task ends and outputs the score to the symptom detection unit 54 or the like.

The video acquisition unit 52 is a processing unit that acquires the video data including the face of the patient who is executing the specific task. For example, the video acquisition unit 52 starts imaging by the imaging unit 13 when the specific task is started, ends the imaging by the imaging unit 13 when the specific task ends, and acquires video data while the specific task is executed, from the imaging unit 13. Then, the video acquisition unit 52 stores the acquired video data in the video data DB 22 and outputs the video data to the AU detection unit 53.

The AU detection unit 53 is a processing unit that detects an occurrence intensity for each AU included in the face of the patient, by inputting the video data acquired by the video acquisition unit 52 into the first machine learning model 23. For example, the AU detection unit 53 extracts each frame from the video data, inputs each frame into the first machine learning model 23, and detects the AU occurrence intensity and the direction of the face of the patient for each frame. Then, the AU detection unit 53 outputs the detected AU occurrence intensity and direction of the face of the patient for each frame, to the symptom detection unit 54. Note that the direction of the face can be specified from the AU occurrence intensity.

The symptom detection unit 54 is a processing unit that detects whether or not a symptom related to the major neurocognitive disorder of the patient occurs, using the temporal change in the occurrence intensity of each AU, the temporal change in the direction of the face of the patient, and the score of the specific task as the feature amounts. For example, the symptom detection unit 54 inputs the "score" acquired by the task execution unit 51, the "temporal change in occurrence intensity of each AU" in which "respective AU occurrence intensities" detected for the respective frames by the AU detection unit are connected in time order, and the "temporal change in direction of face" in which the detected "directions of face" are similarly connected in time order, into the second machine learning model 24, as the feature amounts. Then, the symptom detection unit 54 acquires an output result of the second machine learning model 24 and acquires a higher one of a probability value (reliability) of the occurrence of the symptom and a probability value with no occurrence of the symptom included in the output result, as a detection result. Thereafter, the symptom detection unit 54 displays and outputs the detection result to the display unit 12 and stores the detection result in the storage unit 20.

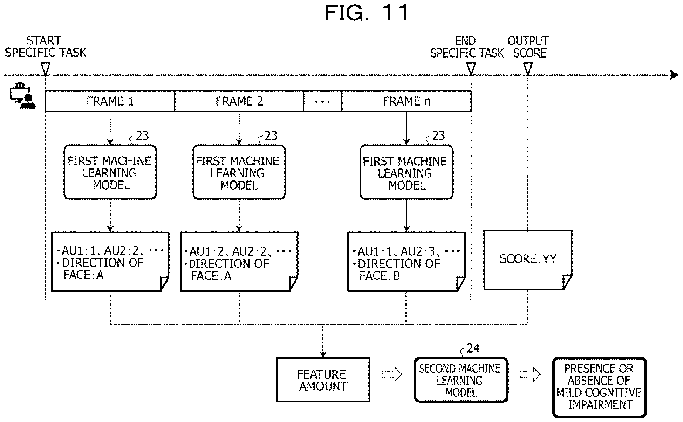

Here, details of the detection of the mild cognitive impairment will be described. FIG. 11 is a diagram for explaining the details of the detection of the mild cognitive impairment. As illustrated in FIG. 11, the operation processing unit 50 acquires video data imaged from start of the specific task to end of the specific task and acquires "occurrence intensity of each AU" and "direction of face" from each frame of the video data.

For example, the operation processing unit 50 inputs image data in a frame 1 into the trained first machine learning model 23 and acquires "AU1 : 2, AU 2 : 5 ..." and "direction of face: A". Similarly, the operation processing unit 50 inputs image data in a frame 2 into the trained first machine learning model 23 and acquires "AU1 : 2, AU 2 : 5 ..." and "direction of face: A". In this way, the operation processing unit 50 specifies the temporal change in each AU of the patient and the temporal change in the direction of the face of the patient, from the video data.

Thereafter, the operation processing unit 50 acquires a score "YY" of the specific task, inputs "temporal change in each AU of patient (AU1 : 2, AU2 : 5, ..., AU1 : 2, AU2 : 5 ...), temporal change in direction of face of patient (direction of face: A, direction of face A,...), and score (YY)" into the second machine learning model 24 as the feature amounts and detects whether or not the mild cognitive impairment occurs.

### <Flow of Preprocessing>

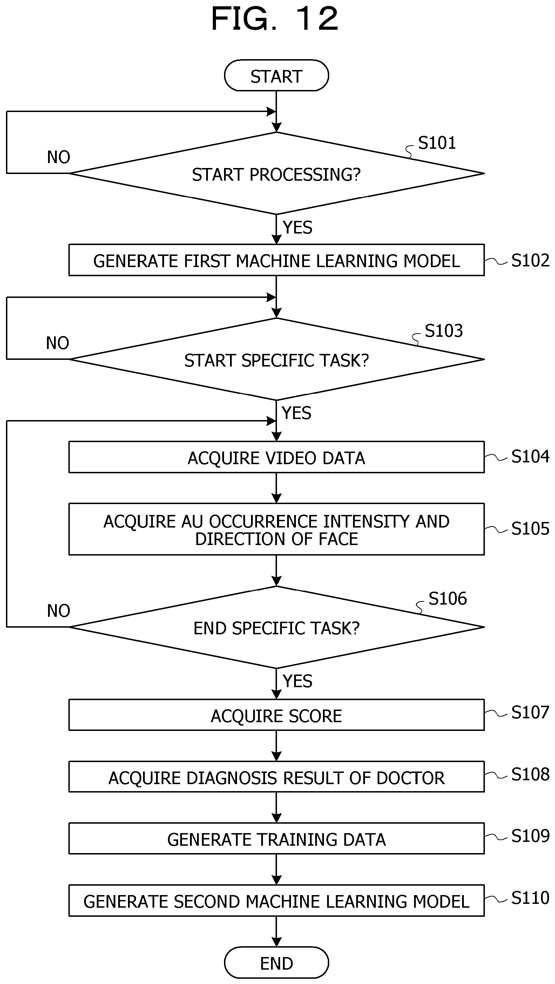

FIG. 12 is a flowchart illustrating a flow of preprocessing. As illustrated in FIG. 12, when processing start is instructed (S101: Yes), the preprocessing unit 40 generates the first machine learning model 23 using the training data (S102).

Subsequently, when the specific task is started (S103: Yes), the preprocessing unit 40 acquires the video data (S104). Then, the preprocessing unit 40 inputs each frame of the video data into the first machine learning model 23 and acquires the occurrence intensity of each AU and the direction of the face, for each frame (S105).

Thereafter, when the specific task ends (S106: Yes), the preprocessing unit 40 acquires the score (S107). Furthermore, the preprocessing unit 40 acquires the diagnosis result of the patient by the doctor (S108).

Then, the preprocessing unit 40 generates the training data including the temporal change in the occurrence intensity of each AU, the temporal change in the direction of the face, and the score (S109) and generates the second machine learning model 24 using the training data (S110).

### <Flow of Detection Processing>

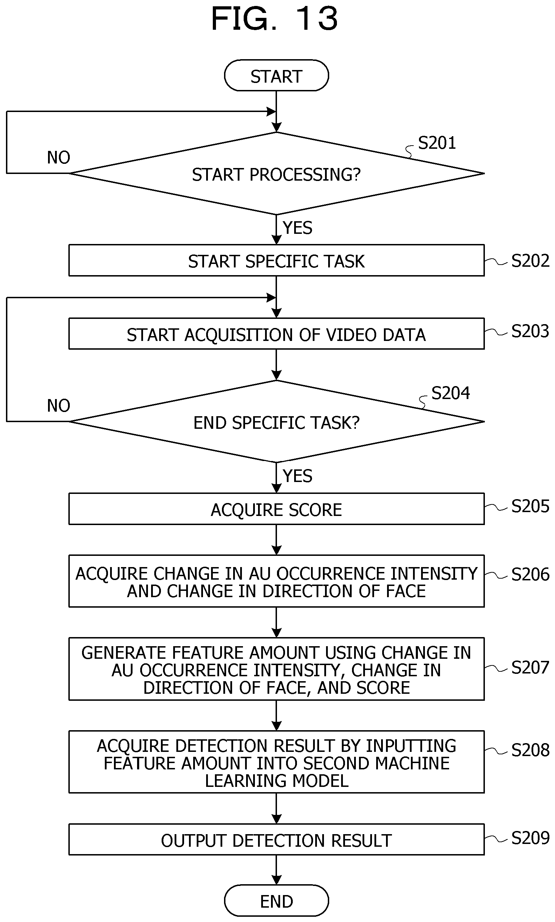

FIG. 13 is a flowchart illustrating a flow of detection processing. As illustrated in FIG. 13, when processing start is instructed (S201: Yes), the operation processing unit 50 executes the specific task on the patient (S202) and starts to acquire the video data (S203).

Then, when the specific task ends (S204: Yes), the operation processing unit 50 acquires the score and ends the acquisition of the video data (S205). The operation processing unit 50 inputs each frame of the video data into the first machine learning model 23 and acquires the occurrence intensity of each AU and the direction of the face, for each frame (S206).

Thereafter, the operation processing unit 50 specifies the temporal change in each AU and the temporal change in the direction of the face, based on the occurrence intensity of each AU and the direction of the face for each frame and generates "temporal change in each AU, temporal change in direction of face, and score" as the feature amounts (S207).

Then, the operation processing unit 50 inputs the feature amount into the second machine learning model 24 and acquires the detection result by the second machine learning model 24 (S208) and outputs the detection result to the display unit 12 or the like (S209).

### <Effects>

As described above, the symptom detection device 10 according to the first embodiment can detect presence or absence of the symptom related to the major neurocognitive disorder, the mild cognitive impairment, or the like, without specialized knowledge of the doctor. Furthermore, the symptom detection device 10 can capture the fine change in the facial expression with a less individual difference, by using the AU and can find the symptom related to the major neurocognitive disorder, the mild cognitive impairment, or the like early.

### [Second Embodiment]

While the embodiment has been described above, the embodiment may be implemented in a variety of different modes in addition to the above-described embodiment.

### (Training Data)

In the above first embodiment, an example has been described in which the temporal change in each AU, the temporal change in the direction of the face, and the score are used as the feature amounts (explanatory variable), as the training data of the second machine learning model 24. However, the present embodiment is not limited to this.

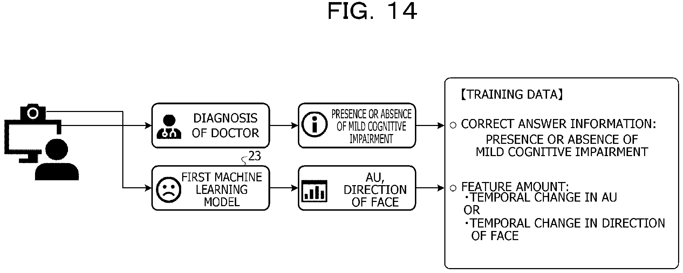

FIG. 14 is a diagram for explaining another example of the training data of the second machine learning model 24. As illustrated in FIG. 14, for example, a symptom detection device 10 may use only the temporal change in each AU as the explanatory variable or may use the temporal change in each AU and the temporal change in the direction of the face as the explanatory variables. Furthermore, although not illustrated, the temporal change in each AU and the score may be used as the explanatory variables.

Furthermore, in the above embodiment, an example has been described in which two values including presence and absence of the symptom of the mild cognitive impairment are used as the objective variable. However, the present embodiment is not limited to this. For example, it is possible to use two values including presence and absence of the symptom of the major neurocognitive disorder as the objective variables, and it is possible to use four values including presence and absence of the symptom of the major neurocognitive disorder and presence and absence of the symptom of the mild cognitive impairment.

In this way, since the symptom detection device 10 can determine the feature amount to be used for training or detection, according to accuracy and cost, it is possible to provide a simple symptom detection service, and it is possible to further provide a detailed service for supporting diagnosis of a doctor.

### (Rule Base)

In the above embodiment, an example has been described in which the presence or absence of the symptom of the mild cognitive impairment is detected using the second machine learning model 24. However, the present embodiment is not limited to this. For example, it is possible to detect the presence or the absence of the symptom of the mild cognitive impairment, using a detection rule in which a pattern of the temporal change in each AU is associated with the presence or the absence of the symptom of the mild cognitive impairment.

Furthermore, the occurrence intensity of each AU can be detected by analyzing video data, other than processing using a first machine learning model 23. For example, it is possible to detect a change in each AU in the entire video data, by setting each AU for a face region of each frame in the video data.

### (Use Form)

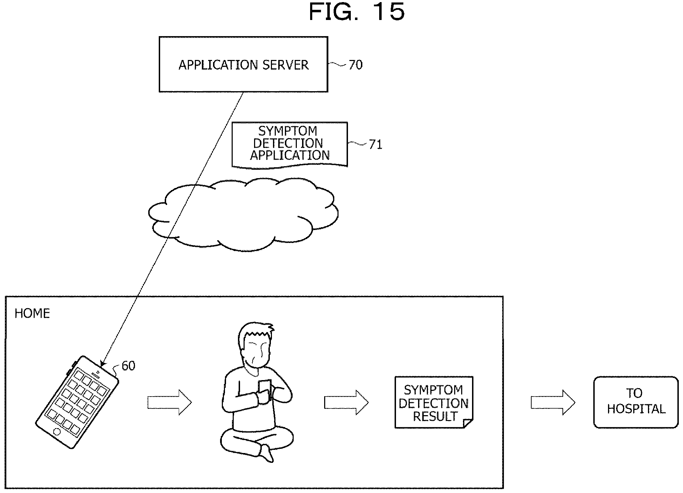

The symptom detection processing described in the first embodiment can be provided to each individual as an application. FIG. 15 is a diagram for explaining an example of a use form of a symptom detection application. As illustrated in FIG. 15, an application server 70 includes the first machine learning model 23 and the second machine learning model 24 trained by a preprocessing unit 40 and holds a symptom detection application (hereinafter, described as application) 71 that executes processing similar to an operation processing unit 50.

In such a situation, a user purchases the application 71 in any place such as home, downloads the application 71 from the application server 70, and installs the application 71 into a smartphone 60 of the user or the like. Then, the user executes the processing similar to the operation processing unit 50 described in the first embodiment, using the smartphone 60 of the user and acquires a detection result of the symptom.

As a result, when the user visits a hospital for examination with a symptom detection result by the application, a hospital side can make the examination in a state where basic detection results have been acquired. Therefore, this is useful for early determination of a disease name or symptom and early start of a treatment.

### (Numerical Values, etc.)

The numerical value example, the training data, the explanatory variable, the objective variable, the number of devices, or the like used in the above embodiments are merely examples and can be arbitrarily changed. In addition, the process flow described in each flowchart may be appropriately modified in a range without inconsistency.

### (System)

Pieces of information including the processing procedure, control procedure, specific names, various types of data and parameters described above or illustrated in the drawings may be altered in any way unless otherwise noted.

In addition, each component of each device illustrated in the drawings is functionally conceptual and does not necessarily have to be physically configured as illustrated in the drawings. In other words, specific forms of distribution and integration of individual devices are not limited to the forms illustrated in the drawings. That is, all or a part thereof may be configured by being functionally or physically distributed or integrated in any units depending on various loads, use situations, or the like. For example, the preprocessing unit 40 and the operation processing unit 50 can be realized by separate devices.

Moreover, all or any part of each processing function performed in each device can be implemented by a central processing unit (CPU) and a program analyzed and executed by the CPU or can be implemented as hardware by wired logic.

### (Hardware)

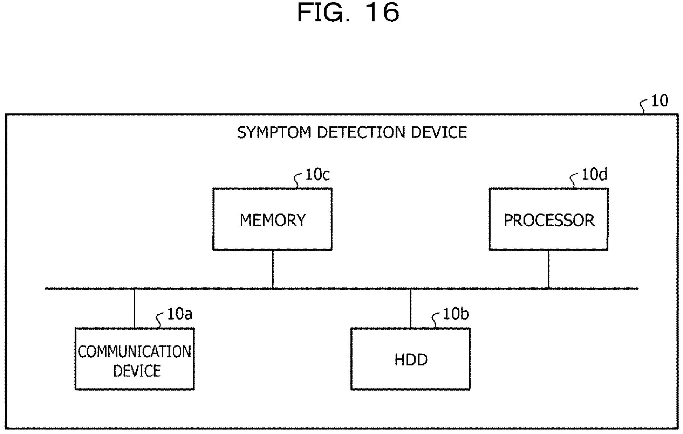

FIG. 16 is a diagram for explaining a hardware configuration example. As illustrated in FIG. 16, the symptom detection device 10 includes a communication device 10a, a hard disk drive (HDD) 10b, a memory 10c, and a processor 10d. Furthermore, the units illustrated in FIG. 16 are coupled to each other by a bus or the like. Note that a display, a touch panel, or the like may be included, in addition to these.

The communication device 10a is a network interface card or the like, and communicates with another device. The HDD 10b stores a program for operating the functions illustrated in FIG. 3, and a DB.

The processor 10d reads, from the HDD 10b or the like, a program that executes processing similar to that of each processing unit illustrated in FIG. 3, and loads it in the memory 10c, thereby operating a process for implementing each function described with reference to FIG. 3 or the like. For example, this process executes functions similar to the functions of each processing unit included in the symptom detection device 10. Specifically, the processor 10d reads a program having functions similar to the functions of the preprocessing unit 40, the operation processing unit 50, and the like from the HDD 10b or the like. Then, the processor 10d executes a process that executes processing similar to the processing of the preprocessing unit 40, the operation processing unit 50, and the like.

In this way, the symptom detection device 10 operates as an information processing device that executes the symptom detection method by reading and executing the program. Furthermore, the symptom detection device 10 can also implement functions similar to the functions of the embodiments described above by reading the program described above from a recording medium by a medium reading device and executing the read program described above. Note that the programs referred to in other embodiments are not limited to being executed by the symptom detection device 10. For example, the embodiments described above may be similarly applied to a case where another computer or server executes the program or a case where these computer and server cooperatively execute the program.

This program may be distributed via a network such as the Internet. In addition, this program may be recorded in a computer-readable recording medium such as a hard disk, a flexible disk (FD), a compact disc read only memory (CD-ROM), a magneto-optical disk (MO), or a digital versatile disc (DVD), and may be executed by being read from the recording medium by a computer.

## Claims

1. A symptom detection program for causing a computer to execute processing comprising:
acquiring video data that includes a face of a patient who is executing a specific task;
detecting each occurrence intensity of each action unit included in the face of the patient, by analyzing the acquired video data; and
detecting a symptom related to a major neurocognitive disorder of the patient, based on a temporal change in the occurrence intensity of each of a plurality of the detected action units.

2. The symptom detection program according to claim 1, wherein
the symptom related to the major neurocognitive disorder of the patient is one of a major neurocognitive disorder or a cognitive impairment.

3. The symptom detection program according to claim 1, for causing the computer to execute processing further comprising:
detecting each occurrence intensity of each action unit included in the face of the patient, by inputting the acquired video data into a first machine learning model.

4. The symptom detection program according to claim 1, for causing the computer to execute processing further comprising:
generating a second machine learning model, by training presence or absence of occurrence of the symptom related to the major neurocognitive disorder of the patient, by using the temporal change in the occurrence intensity of each of the plurality of action units as a feature amount.

5. The symptom detection program according to claim 1, for causing the computer to execute processing further comprising:
generating a second machine learning model, by training presence or absence of occurrence of the symptom related to the major neurocognitive disorder of the patient, by using the temporal change in the occurrence intensity of each of the plurality of action units and a temporal change in a direction of the face of the patient as feature amounts.

6. The symptom detection program according to claim 1, wherein
the specific task is an application or an interactive application that applies a load on a cognitive function and examines the cognitive function.

7. The symptom detection program according to claim 1 or 6, for causing the computer to execute processing further comprising:
acquiring a score of the specific task; and
generating a second machine learning model, by training presence or absence of occurrence of the symptom related to the major neurocognitive disorder of the patient, by using the temporal change in the occurrence intensity of each of the plurality of action units, a temporal change in a direction of the face of the patient, and the score of the specific task as feature amounts.

8. A symptom detection method implemented by a computer, the symptom detection method comprising:
acquiring video data that includes a face of a patient who is executing a specific task;
detecting each occurrence intensity of each action unit included in the face of the patient, by analyzing the acquired video data; and
detecting a symptom related to a major neurocognitive disorder of the patient, based on a temporal change in the occurrence intensity of each of a plurality of the detected action units.

9. A symptom detection device comprising:
a processor configured to:
acquire video data that includes a face of a patient who is executing a specific task;
detect each occurrence intensity of each action unit included in the face of the patient, by analyzing the acquired video data; and
detect a symptom related to a major neurocognitive disorder of the patient, based on a temporal change in the occurrence intensity of each of a plurality of the detected action units.
